# EUROPEAN PATENT APPLICATION

(11) **EP 3 992 978 A1**
(43) Date of publication of application: **04.05.2022**
(21) Application number: 21205131.2
(22) Date of filing: 27.10.2021
(51) Int. Cl.: G16H 20/40, G16H 30/40, G16H 50/20

(54) **KNEE ARTHROPLASTY FUNCTIONAL DIGITAL TWIN**

(30) Priority: 27.10.2020 US 202063106171 P; 16.03.2021 US 202163161787 P
(71) Applicant: Zimmer, Inc., Warsaw, IN 46580 (US)
(72) Inventor: OAK, Varun Jayant, 576149 Singapore (SG)
(74) Representative: Mays, Julie

(57) **Abstract**

The present disclosure describes technical solutions to various technical problems facing knee surgery surgical procedures. In an embodiment, this solution includes prediction of postoperative knee functionality by creating a functional digital twin computer-based model of the patient knee joint. The functional digital twin may be based on medical imagery and preoperative joint sensor data, such as motion and position data. This functional digital twin is used for digital mirroring of the knee joint functionality, and this digital mirroring enables the surgeon to determine knee functionality before and after planned surgical bone cuts, soft tissue releases, or other surgical procedure steps. This digital mirroring is performed preoperatively to determine an optimal set of surgical procedures, which improves the patient's satisfaction in the functional outcome by reducing or eliminating the surgeon-specific subjectivity and intraoperative trial-and-error approaches.

## Description

### CLAIM OF PRIORITY

This application claims the benefit of priority to U.S. Provisional Application Serial No. 63/106,171, filed October 27, 2020, titled "Knee Arthroplasty Functional Digital Twin," and to U.S. Provisional Application Serial No. 63/161,787, filed March 16, 2021, titled "Knee Arthroplasty Functional Digital Twin," which are hereby incorporated herein by reference in their entirety.

### FIELD

The present application relates to computer modeling of a knee joint.

### BACKGROUND

Orthopedic surgeons currently use traditional instruments to perform knee replacement surgery (e.g., knee arthroplasty). The surgeon uses surgical experience and judgement to perform bone cuts and ligament releases based on his training, surgical skills, and experience. The surgeon aims at achieving post-surgical knee alignments that are closest to a guidance range for the population rather than customizing these measurements for each patient.

While surgeons achieve the expected post-operative results in many cases, a significant number of patients remain dissatisfied post-operatively after a total knee replacement. This dissatisfaction happens mainly due to inadequate post-operative pain relief and lack of functional improvement. The major factors that lead to this dissatisfaction include:
1. Faulty implant positioning due to improper technique or inadequate planning.
2. A lack of return of the knee function and movement as expected by the patient.
3. Excessive soft tissue releases causing prolonged knee pain.
4. Faulty implant sizing resulting in restricted function, increased wear on the implant, and reduced implant life.
5. A one-size-fits-all approach in total knee replacement that does not take into consideration preoperative patient function.
6. A trial-and-error approach that has high degree of surgeon-specific subjectivity and variability. Performing intraoperative implant trials after performing bone cuts and using physical trial implants. The surgeon performs corrective ligament releases or additional bone resections in order to fit the implant perfectly within the knee.
7. Surgeon's inability to assess knee behavior and proprioceptive responses in real time from active muscle and soft-tissue engagement. Assessing the patient's knee intraoperatively when the patient is under anesthesia. The surgeon assesses the patient's knee intraoperatively when the knee is not under active muscle tension or under the effect of gravity and patient movements.

However, a surgeon has limited ability to assess the patient's knee pre-operatively to assess the baseline mechanics for that particular patient. The surgeon may not be able to plan the surgical cuts and ligament releases pre-operatively, and may rely on intra-operative trial and error. The surgeon may also have to rely on postoperative assessments to determine functional outcomes with the desired knee implants.

### BRIEF DESCRIPTION OF THE DRAWINGS

FIG. 1 is a diagram of a digital twin sensor array, in accordance with some embodiments.
FIG. 2 is a diagram of a lower limb functional cross-sectional map, in accordance with some embodiments.
FIG. 3 is a diagram of a 3D limb model, in accordance with some embodiments.
FIG. 4 is a diagram of a functional digital model, in accordance with some embodiments.
FIG. 5 is a diagram of a patient knee digital twin, in accordance with some embodiments.
FIG. 6 illustrates a flow chart showing method of generating and applying of a functional digital twin model, in accordance with some embodiments.
FIG. 7 illustrates an example of a block diagram of a machine upon which any one or more of the techniques discussed herein may perform in accordance with some embodiments.

### DETAILED DESCRIPTION

The present disclosure describes technical solutions to various technical problems facing knee surgery surgical procedures. In an embodiment, a solution includes prediction of pre-operative or post-operative knee functionality (e.g., biomechanical knee joint movements, range of motion) by creating a computer-based model of the patient knee joint. This computer-based model of the knee joint is referred to herein as a functional digital twin. The functional digital twin of one knee of the patient may also be compared with a similar functional digital twin (e.g., digital mirroring model) of the contralateral knee. This digital mirroring technique enables the surgeon to determine the knee functionality pre-operatively to plan surgical bone cuts, soft tissue releases, or other surgical procedure steps by comparing with the other knee which would set the baseline for the patient. This digital mirroring is performed preoperatively to determine an optimal set of surgical procedures, thereby reducing or eliminating intraoperative trial-and-error and patient postoperative dissatisfaction. This digital mirroring may also be used to analyze and compare both patient knees, such as when an osteoarthritic knee is compared to the healthier contralateral knee to determine healthy biomechanical knee joint movements.

The generation of the functional digital twin may be based on preoperative sensor data used to characterize the patient knee, and optionally additional sensor data from other joints such as patient's hip or ankle. The sensors may include wearable sensors attached to predetermined positions on the patient, such as various wearable sensor bands sized to attach to a location proximate to the patient knee, ankle, and hip. The sensors may include other wearable sensors, such as a smartwatch, knee brace, shoe inserts, or other sensors. The wearable sensors may include one or more pressure sensors, motion sensors, gyroscopes, accelerometers, absolute location sensors, intra-sensor location sensors, strain sensors, or other sensors. The sensor data may include patient imaging data captured through medical imagery (e.g., computed tomography (CT), magnetic resonance imaging (MRI), fluoroscopic motion capture or optical imagery (e.g., motion capture device). The medical imagery may be collected during the initial osteoarthritis diagnosis or in preoperative planning, and may include capturing x-rays in anteroposterior view, lateral view, skyline view, or other views. In an example, a series of preoperative static bone medical imagery may be combined with dynamic fluoroscopic motion capture and patient sensor data to generate the functional digital twin. Machine Learning (ML) may be applied to the sensor data, such as to generate, validate, or improve the functional digital twin. In an example, a previously trained ML model may be applied to the sensor data to generate the functional digital twin. In another example, the sensor data may be used to train the ML model.

The functional digital twin provides various features for preoperative planning of the surgical procedure. The functional digital twin provides the surgeon with a moveable model of the patient knee, which the surgeon may use to analyze how the knee moves through various ranges of motion in the preoperative natural state. This may be used to set a baseline on the patient's current gait and kinematics and how the patient's body has adapted to movements due to osteoarthritis. The functional digital twin uses the sensor-based data to provide 3D map of how patient's knees move, linking the physical and virtual knees of the patient. The functional digital twin may be used to analyze movement of various anatomical references in the patient's joint. The anatomical references may include the medial collateral ligament (MCL), lateral collateral ligament (LCL), anterior cruciate ligament (ACL), posterior cruciate ligament (PCL), bone surfaces, capsule, menisci, bony osteophytes, or other anatomical references. The surgeon may perform various virtual bone cuts and soft tissue releases on the digital model, and generate predictions of how each surgical procedure step would affect the patient post-operative knee functionality. The effect of each surgical procedure step may be determined through analysis of historical surgical procedure data. For example, if the surgeon resects 3 mm of the distal femur medial epicondyle and takes 2 mm of the MCL, the patient knee hip-knee-ankle (HKA) axis would be altered by X degrees, the MCL and capsule would be stressed by Y%, through Z degrees of functional range of motion. In an example, the effect of each surgical procedure step may be determined or verified using ML, such as by applying a previously trained ML model to the functional digital twin and the virtual surgical steps. Each of the virtual surgical steps may be performed on the patient-specific functional digital model to determine functional outcomes, thereby avoiding an intraoperative trial-and-error approach. The surgeon can also develop a similar functional digital twin of the opposite knee (assuming the other knee is normal) and perform all cuts and resections to match the exact movement of the other knee. This feature is called as digital mirroring.

The functional digital twin provides various advantages compared to alternative procedures. Compared to preoperative planning using only 2D x-ray imagery that stitches hip, knee, and ankle x-ray imagery to determine the HKA axis, the use of a functional digital twin generates a reliable HKA axis. In contrast with a use of 2D x-ray imagery and generalized computer-aided templating that estimates one or more surgical implant sizes that may be tested intraoperatively, the functional digital twin may be used preoperatively to test and select a surgical implant. In contrast with each of these alternative techniques whose outcomes depend largely on whether the surgeon is subjectively satisfied with the intraoperative knee functionality, the functional digital twin improves the patient's satisfaction in the functional outcome by reducing or eliminating the surgeon-specific subjectivity and intraoperative trial-and-error approaches. This also reduces the inventory in the implant hospital as the surgeon will be able to precisely pick the exact size of the implant pre-operatively and only order those sizes for the patient.

The various features of the functional digital twin result in an improved or optimized surgical plan. The preoperative experimentation using the functional digital twin enables the surgeon to predict and prepare preoperatively for the optimal functional outcome. This allows the surgeon to simulate complex, risky, and crucial surgical steps on the patient's digital knee model while avoiding intraoperative trial-and-error on the patient, such as by reducing number of surgical steps, reducing operating time, and improving procedure reliability. The use of the functional digital twin may significantly improve patient satisfaction, as the digital model may be used to determine exactly how the patient will walk postoperatively. Because the functional digital twin improves surgical outcomes based on patient-based baseline sensor data and simulates patient-specific functional outcomes, this significantly improves the ability to set and meet or exceed patient expectations.

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.

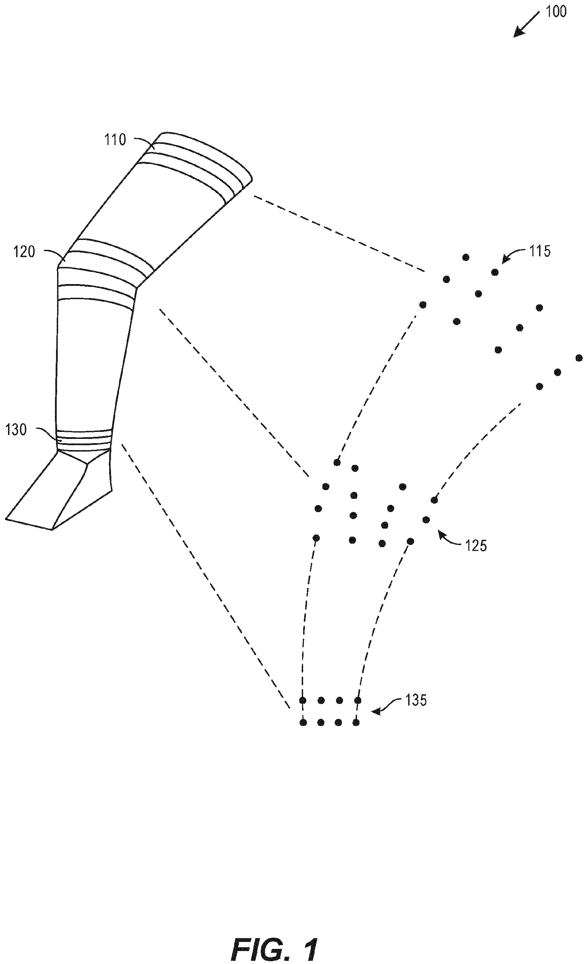

FIG. 1 is a diagram of a digital twin sensor array 100, in accordance with some embodiments. Sensory array may include one or more sensor bands, such as an upper-thigh sensor band 110, a knee sensor band 120, and an ankle sensor band 130. Each sensor band may include an array of sensors, such as upper-thigh sensor array 115, knee sensor array 125, and ankle sensor array 135. In an example, the sensor band may include a silicon band or flexible tape with embedded sensors. The silicon band or flexible tape may be composed of a radiopaque material so that medical imagery may be used to capture the arrangement of each sensor array while minimizing or eliminating visibility of the radiopaque material.

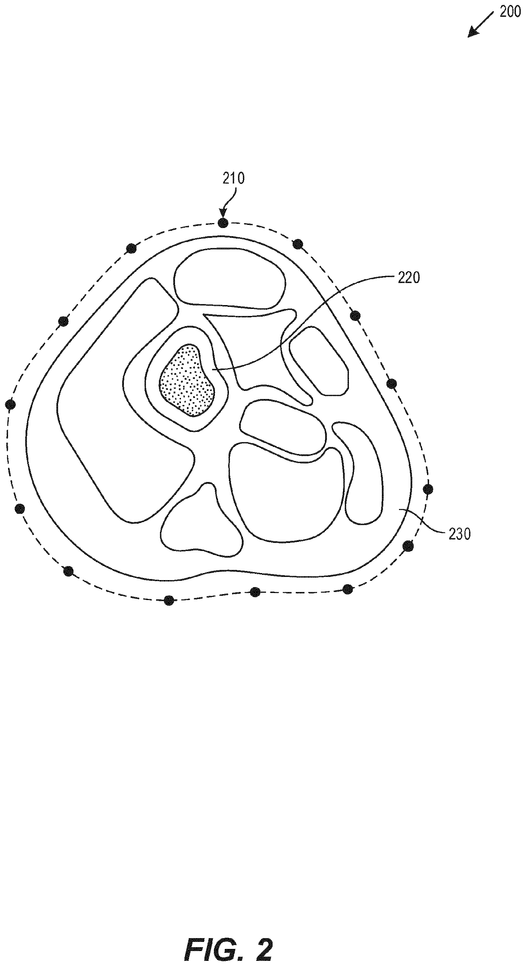

The sensors may include piezo sensors, such as piezoelectric sensors, piezoresistive sensors, carbon nanotube-based sensors, silver nanoparticle sensors, dielectric material sensors, or other sensors. The sensors may be joined through one or more power or communication lines, and one or more of the sensors may include autonomous power, memory, or communication capabilities. The sensors may be used to characterize the joint, such as by sensing position, motion, strain, torque, torsion, or other joint sensor information. The sensors may be joined through one or more tensile or compressive members, such as to improve detection of strain or positional displacement. Whenever the patient moves each joint, these sensors detect micro motions of the joints in 3D space. The sensors may store data internally and includes a communication port for data retrieval, or may communicate with other sensors or with a communication device using a communication network (e.g., Bluetooth, NFC). The sensor data may be used to generate a functional map, such as shown in FIG. 2.

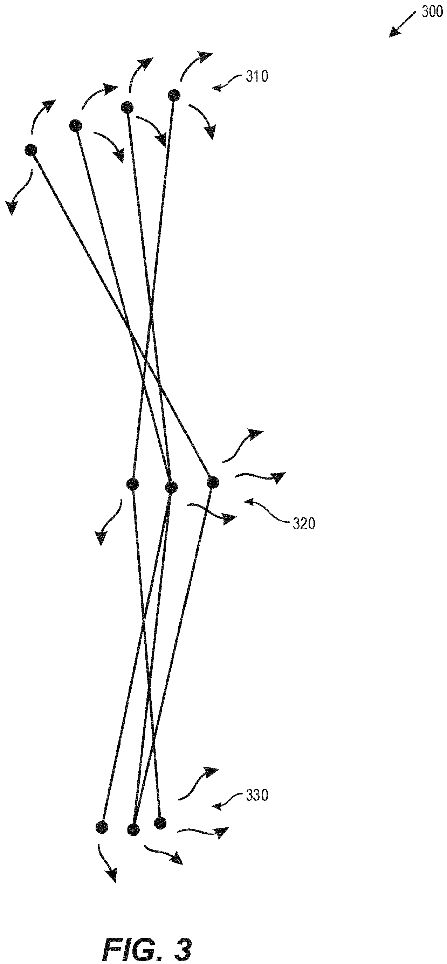

FIG. 2 is a diagram of a lower limb functional cross-sectional map 200, in accordance with some embodiments. Functional map 200 shows a band of sensors 210 positioned around a cross-sectional view of a knee. The cross-sectional view may include one or more bony structures 220 and surrounding soft tissue 230, which may be captured during preoperative medical imaging. Functional map 200 may be used to show how each sensor point moves throughout various joint movements, and may be used to show relative positioning with each other over a defined period of time. For example, the bony structures 220 and surrounding soft tissue 230 may change the position, strain, and other data gathered by the band of sensors 210. Functional maps may be generated for various patient positions, motions, or activities to generate a full limb model, such as shown in FIG. 3.

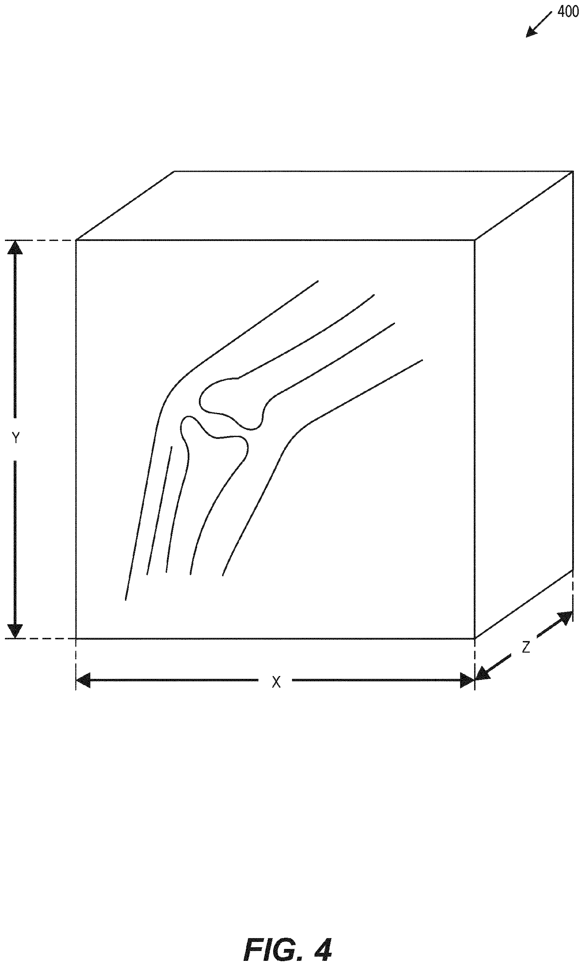

FIG. 3 is a diagram of a 3D limb model 300, in accordance with some embodiments. One or more CT and MRI scans of the patient may be performed with the sensors on the patient. The sensor arrays may include a hip sensor array 310, a knee sensor array 320, and an ankle sensor array 330. The sensor arrays may be used to capture absolute and relative sensor data, such as sensor position and motion relative to other sensors. The CT and MRI scans may provide multiple static snapshots of the joint anatomy and sensor arrays, whereas the sensor data points may be captured continually over a period of time. The sensor data points may be collected in real time, and may be matched with the sensors points in the medical CT and MRI scans, to generate the functional digital model shown in FIG. 4.

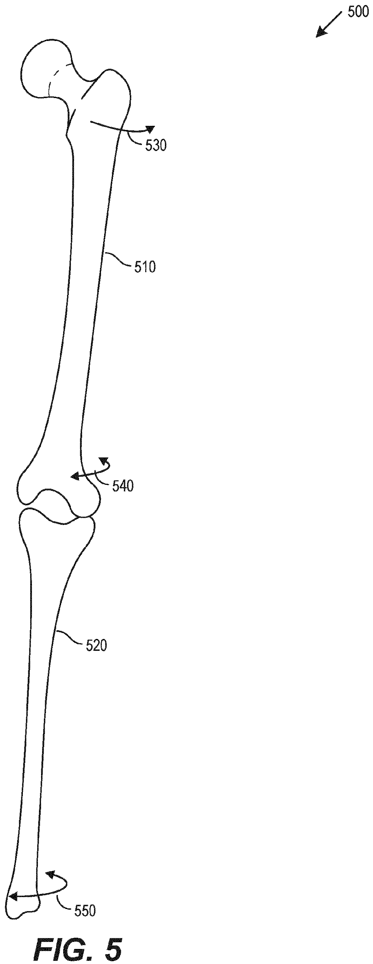

FIG. 4 is a diagram of a functional digital model 400, in accordance with some embodiments. The functional digital model 400 provides the ability to simulate joint motion, such as by simulating a 3D model based on patient medical imagery and sensor data. For patient positions or motions that are not captured in the in the medical CT and MRI scans, the sensor data points may be extrapolated or interpolated to generate additional static or dynamic 3D limb model positions or motions, such as to simulate the motion of the patient's CT and MRI image as if the patient were actually walking or to indicate the full range of motion of the patient knee. In an example, a previously trained ML model may be applied to the sensor data to generate the additional static or dynamic 3D limb model positions or motions, or to create a moving functional CT and MRI image. The medical imagery, sensor data, and generated static or dynamic 3D limb model positions or motions may be combined to generate the functional digital model 400. The functional digital model 400 may be used to generate a simulated patient knee digital twin 500, such as shown in FIG. 5.

FIG. 5 is a diagram of a patient knee digital twin 500, in accordance with some embodiments. The digital twin 500 may include one or more long bones proximate to the joint, such as the femur 510 and the tibia 520. The digital twin 500 may be used in a surgical planning tool. The surgical planning tool may allow the surgeon to change position or simulate motion, such as a rotation of the upper femur 510, a rotation of the lower femur 540, a rotation of the lower tibia 520, or other motions or positions. The digital twin 500 uses the functional digital model to simulate and display the effect of various surgical procedures. The surgical planning tool may allow the surgeon to simulate various bone cuts or soft tissue releases on the digital twin 500 preoperatively and generate a simulated resulting change in the joint. For example, a surgeon may simulate cutting 3 mm of distal medial condyle and a 50% lateral capsule release, and the digital twin may indicate the gap will be changed by X mm and the postoperative function will be affected by Y% compared with preoperative function.

The surgeon may experiment with various cuts, releases, motion simulations, or other features simulated by the digital twin 500, and then select the surgical procedures that correspond with the best functional outcomes for patient limb function and movement. The surgeon may also set the outcome based on functional digital mirroring thereby comparing the arthritic knee model with the model of the other knee of the patient. In an example, ML or other surgical analytics may use the digital twin 500 to improve surgical procedure selection, such as by recommending a surgical procedure to improve a patient outcome, by providing an estimated resulting range of motion, or by recommending one or more surgical procedure steps to accompany a selected surgical procedure step. The surgical plan may be generated, modified, or executed based on a planned use of a robotic surgical device. For example, bone cuts and other procedures specific to a robotic surgical device may be selected and loaded into the robotic surgical device, the robotic surgical device performs the bone cuts in the patient according to the surgical plan, and the surgeon performs the soft tissue releases as planned using the digital twin 500. The surgical plan may include a sequence of local robotic surgical steps or telerobotic surgical steps, where the steps may be based on steps performed by the surgeon in a virtual surgery on the patient's functional knee model. The local robotic surgical steps or telerobotic surgical steps may enable the robotic surgical device to perform the entire surgery by itself or under the control or supervision of a surgeon. This may allow the robot to function autonomously in any location of the world, where the robotic surgical steps may be generated based on surgical steps generated by a surgeon located remotely from the robotic surgical device.

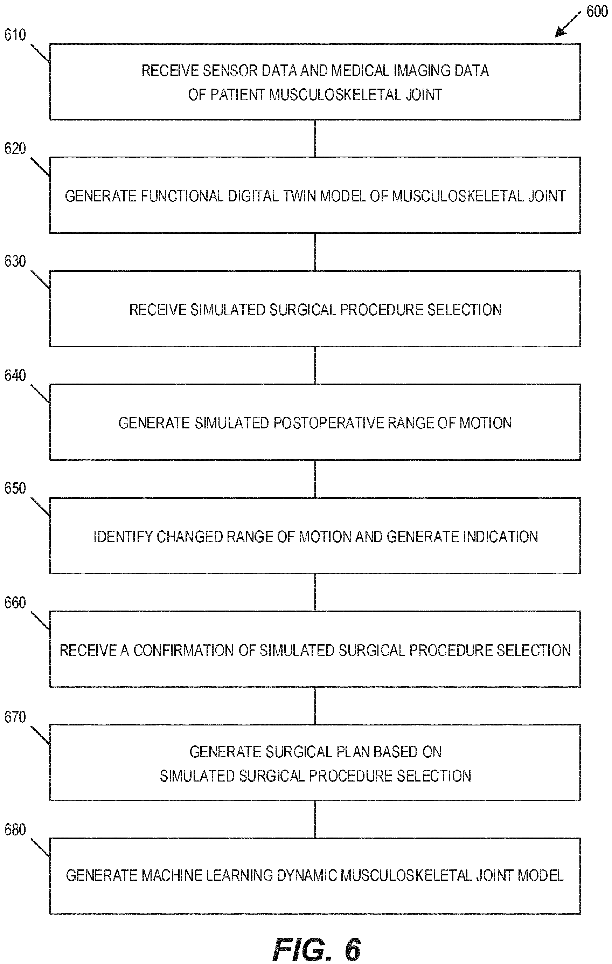

FIG. 6 illustrates a flow chart showing method 600 of generating and applying of a functional digital twin model, in accordance with some embodiments. Method 600 includes receiving 610 sensor data and medical imaging data of a musculoskeletal joint of a patient. Method 600 includes generating 620 a functional digital twin model of the musculoskeletal joint based on the received sensor data and the received medical imaging data. The functional digital twin model may indicate a preoperative range of motion of the musculoskeletal joint.

In an embodiment, the musculoskeletal joint includes a knee of the patient. The plurality of sensors may be attached around the knee of the patient, around a hip of the patient, and around an ankle of the patient. The plurality of sensors may include a plurality of piezo sensors. The plurality of sensors may be used to sense at least one of musculoskeletal strain, musculoskeletal torque, and musculoskeletal torsion. The received sensor data may include a relative position of each of the plurality of sensors, and may include motion data captured during a movement of the musculoskeletal joint.

In an embodiment, the medical imaging data includes a plurality of images of the musculoskeletal joint and the plurality of sensors. The plurality of sensors may be attached to a plurality of positions on the patient relative to the musculoskeletal joint, and may be embedded in a flexible ring fixed around the musculoskeletal joint. The flexible ring may be formed using a radiopaque material. The radiopaque material may reduce or eliminate the visibility of the ring in the medical imaging data while allowing the sensors to remain visible in the medical imaging data. The medical imaging data of the musculoskeletal joint may include a plurality of static musculoskeletal joint positions. The generation of the functional digital twin model may include generating a functional motion model, the functional motion model simulating a dynamic musculoskeletal joint motion.

In an embodiment, method 600 includes receiving 630 a simulated surgical procedure selection. The simulated surgical procedure selection may include at least one of a soft tissue release and a bone cut. Method 600 includes generating 640 a simulated postoperative range of motion of the musculoskeletal joint. The simulated postoperative range of motion may be based on the received simulated surgical procedure selection and the generated functional digital twin model.

In an embodiment, method 600 includes identifying 650 a changed range of motion based on the received simulated soft tissue release input and generating an indication of the changed range of motion.

In an embodiment, method 600 includes receiving 660 a confirmation of the simulated surgical procedure selection and generating 670 a surgical plan based on the generated functional digital twin model and the confirmation of the simulated surgical procedure selection. The surgical plan may include a plurality of robotic surgical steps and a plurality of surgeon surgical steps. The robotic surgical steps may include a plurality of bone cut surgical steps. The plurality of surgeon surgical steps may include a plurality of soft tissue releases.

Method 600 may include generating 680 a machine learning dynamic musculoskeletal joint model based on the received sensor data, the received receiving medical imaging data, and a plurality of musculoskeletal joint machine learning data. The generation 620 of the functional digital twin model may be based on the generated machine learning dynamic musculoskeletal joint model. The generation of the motion model may include generating a plurality of dynamic musculoskeletal joint data based on the generated machine learning dynamic musculoskeletal joint model, the received sensor data, and the received receiving medical imaging data.

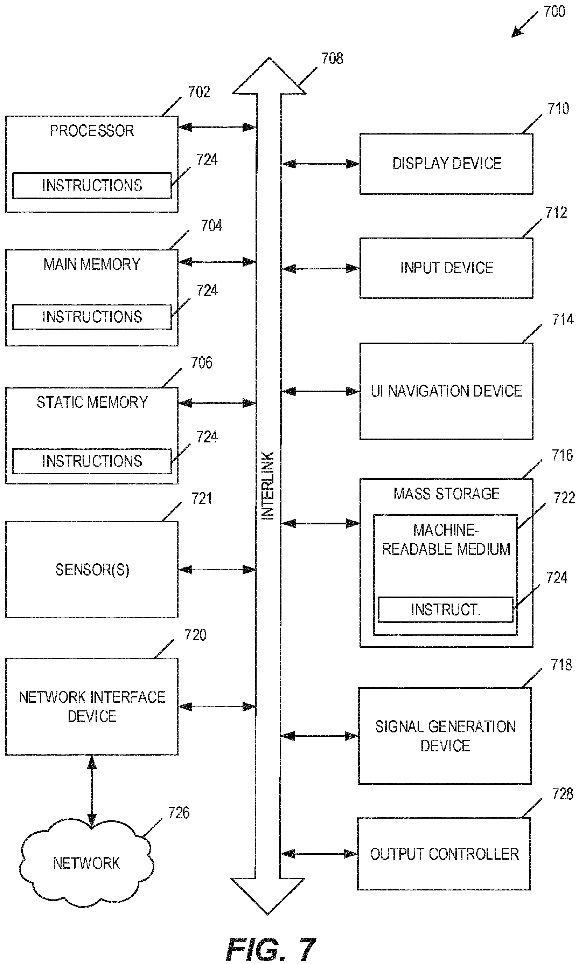

FIG. 7 illustrates an example of a block diagram of a machine 700 upon which any one or more of the techniques (e.g., methodologies) discussed herein may perform in accordance with some embodiments. In alternative embodiments, the machine 700 may operate as a standalone device or may be connected (e.g., networked) to other machines. In a networked deployment, the machine 700 may operate in the capacity of a server machine, a client machine, or both in server-client network environments. The machine 700 may be a personal computer (PC), a tablet PC, a personal digital assistant (PDA), a mobile telephone, a web appliance, a network router, switch or bridge, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein, such as cloud computing, software as a service (SaaS), other computer cluster configurations.

Examples, as described herein, may include, or may operate on, logic or a number of components, modules, or like mechanisms. Such mechanisms are tangible entities (e.g., hardware) capable of performing specified operations when operating. In an example, the hardware may be specifically configured to carry out a specific operation (e.g., hardwired). In an example, the hardware may include configurable execution units (e.g., transistors, circuitry, etc.) and a computer readable medium containing instructions, where the instructions configure the execution units to carry out a specific operation when in operation. The configuring may occur under the direction of the execution units or a loading mechanism. Accordingly, the execution units are communicatively coupled to the computer readable medium when the device is operating. For example, under operation, the execution units may be configured by a first set of instructions to implement a first set of features at one point in time and reconfigured by a second set of instructions to implement a second set of features.

Machine (e.g., computer system) 700 may include a hardware processor 702 (e.g., a central processing unit (CPU), a graphics processing unit (GPU), a hardware processor core, processing circuitry, or any combination thereof), a main memory 704 and a static memory 706, some or all of which may communicate with each other via an interlink (e.g., bus) 708. The machine 700 may further include a display unit 710, an alphanumeric input device 712 (e.g., a keyboard), and a user interface (UI) navigation device 714 (e.g., a mouse). In an example, the display unit 710, alphanumeric input device 712 and UI navigation device 714 may be a touch screen display. The display unit 710 may include goggles, glasses, an augmented reality (AR) display, a virtual reality (VR) display, or another display component. For example, the display unit may be worn on a head of a user and may provide a heads-up-display to the user. The alphanumeric input device 712 may include a virtual keyboard (e.g., a keyboard displayed virtually in a VR or AR setting.

The machine 700 may additionally include a storage device (e.g., drive unit) 716, a signal generation device 718 (e.g., a speaker), a network interface device 720, and one or more sensors 721, such as a global positioning system (GPS) sensor, compass, accelerometer, or other sensor. The machine 700 may include an output controller 728, such as a serial (e.g., universal serial bus (USB), parallel, or other wired or wireless (e.g., infrared (IR), near field communication (NFC), etc.) connection to communicate or control one or more peripheral devices.

The storage device 716 may include a machine readable medium 722 that is non-transitory on which is stored one or more sets of data structures or instructions 724 (e.g., software) embodying or utilized by any one or more of the techniques or functions described herein. The instructions 724 may also reside, completely or at least partially, within the main memory 704, within static memory 706, or within the hardware processor 702 during execution thereof by the machine 700. In an example, one or any combination of the hardware processor 702, the main memory 704, the static memory 706, or the storage device 716 may constitute machine readable media.

While the machine readable medium 722 is illustrated as a single medium, the term "machine readable medium" may include a single medium or multiple media (e.g., a centralized or distributed database, or associated caches and servers) configured to store the one or more instructions 724.

The term "machine readable medium" may include any medium that is capable of storing, encoding, or carrying instructions for execution by the machine 700 and that cause the machine 700 to perform any one or more of the techniques of the present disclosure, or that is capable of storing, encoding or carrying data structures used by or associated with such instructions. Non-limiting machine readable medium examples may include solid-state memories, and optical and magnetic media. Specific examples of machine readable media may include: non-volatile memory, such as semiconductor memory devices (e.g., Electrically Programmable Read-Only Memory (EPROM), Electrically Erasable Programmable Read-Only Memory (EEPROM)) and flash memory devices; magnetic disks, such as internal hard disks and removable disks; magneto-optical disks; and CD-ROM and DVD-ROM disks.

The instructions 724 may further be transmitted or received over a communications network 726 using a transmission medium via the network interface device 720 utilizing any one of a number of transfer protocols (e.g., frame relay, internet protocol (IP), transmission control protocol (TCP), user datagram protocol (UDP), hypertext transfer protocol (HTTP), etc.). Example communication networks may include a local area network (LAN), a wide area network (WAN), a packet data network (e.g., the Internet), mobile telephone networks (e.g., cellular networks), Plain Old Telephone (POTS) networks, and wireless data networks (e.g., Institute of Electrical and Electronics Engineers (IEEE) 802.11 family of standards known as Wi-Fi^{®}, as the personal area network family of standards known as Bluetooth^{®} that are promulgated by the Bluetooth Special Interest Group, peer-to-peer (P2P) networks, among others. In an example, the network interface device 720 may include one or more physical jacks (e.g., Ethernet, coaxial, or phone jacks) or one or more antennas to connect to the communications network 726. In an example, the network interface device 720 may include a plurality of antennas to wirelessly communicate using at least one of single-input multiple-output (SIMO), multiple-input multiple-output (MIMO), or multiple-input single-output (MISO) techniques. The term "transmission medium" shall be taken to include any intangible medium that is capable of storing, encoding, or carrying instructions for execution by the machine 700, and includes digital or analog communications signals or other intangible medium to facilitate communication of such software.

Each of these non-limiting examples may stand on its own, or may be combined in various permutations or combinations with one or more of the other examples.

Example 1 is a method for generating an arthroplasty functional digital twin, the method comprising: receiving first sensor data from a first plurality of sensors attached to a patient, the first sensor data characterizing a first musculoskeletal joint of the patient; receiving medical imaging data of the first musculoskeletal joint; generating, at a processing circuitry of a device, a functional digital twin model of the first musculoskeletal joint based on the first sensor data and the medical imaging data, the functional digital twin model indicating a preoperative range of motion of the first musculoskeletal joint; and outputting the functional digital twin model of the first musculoskeletal joint.

In Example 2, the subject matter of Example 1 includes, receiving second sensor data from a second plurality of sensors attached to the patient, the second sensor data characterizing a second musculoskeletal joint of the patient, wherein the second musculoskeletal joint is contralateral to the first musculoskeletal joint; and generating a digital mirroring model of the second musculoskeletal joint based on the second sensor data, the digital mirroring model indicating a baseline range of motion of the second musculoskeletal joint.

In Example 3, the subject matter of Example 2 includes, generating a simulated digital mirroring range of motion of the first musculoskeletal joint based on the digital mirroring model.

In Example 4, the subject matter of Examples 2-3 includes, wherein the first sensor data characterizing the first musculoskeletal joint includes determining at least one of a joint position, a joint motion, a joint strain, a joint torque, or a joint torsion.

In Example 5, the subject matter of Examples 1-4 includes, receiving a simulated surgical procedure selection, the simulated surgical procedure selection including at least one of a soft tissue release and a bone cut; generating a predicted range of motion of the first musculoskeletal joint based on the simulated surgical procedure selection and the functional digital twin model; and generating an indication of the predicted range of motion.

In Example 6, the subject matter of Example 5 includes, receiving a confirmation of the simulated surgical procedure selection; and generating a surgical plan based on the functional digital twin model and the confirmation of the simulated surgical procedure selection.

In Example 7, the subject matter of Example 6 includes, wherein the surgical plan includes a plurality of robotic surgical steps.

In Example 8, the subject matter of Examples 6-7 includes, wherein: the surgical plan includes a plurality of telerobotic surgical steps; and the surgical plan includes a plurality of surgeon control steps, the plurality of surgeon control steps controlling a telerobotic sequence of the plurality of telerobotic surgical steps.

In Example 9, the subject matter of Examples 6-8 includes, wherein: the surgical plan includes a plurality of robotic surgical steps and a plurality of surgeon surgical steps; the plurality of surgeon surgical steps includes a plurality of soft tissue releases; and the plurality of robotic surgical steps includes a plurality of bone cut surgical steps.

In Example 10, the subject matter of Examples 1-9 includes, wherein the medical imaging data includes a plurality of images of the first musculoskeletal joint and the first plurality of sensors.

In Example 11, the subject matter of Examples 1-10 includes, wherein the first plurality of sensors is attached to a plurality of predetermined positions on the patient, the plurality of predetermined positions on the patient selected to characterize the first musculoskeletal joint.

In Example 12, the subject matter of Examples 1-11 includes, wherein: the first plurality of sensors is embedded in a flexible ring fixed around the first musculoskeletal joint; the flexible ring is formed using a radiopaque material; and the first plurality of sensors is visible in the medical imaging data.

In Example 13, the subject matter of Examples 1-12 includes, wherein: the first musculoskeletal joint includes a knee of the patient; and the first plurality of sensors is attached around the knee of the patient, around a hip of the patient, and around an ankle of the patient.

In Example 14, the subject matter of Examples 1-13 includes, wherein: the first plurality of sensors includes a plurality of piezo sensors; and the first plurality of sensors senses at least one of musculoskeletal strain, musculoskeletal torque, and musculoskeletal torsion.

In Example 15, the subject matter of Examples 1-14 includes, wherein the first sensor data includes a relative position of each of the first plurality of sensors.

In Example 16, the subject matter of Examples 1-15 includes, wherein the first sensor data includes motion data captured during a movement of the first musculoskeletal joint.

In Example 17, the subject matter of Examples 1-16 includes, wherein: the medical imaging data of the first musculoskeletal joint includes a plurality of static musculoskeletal joint positions; and generating the functional digital twin model includes generating a functional motion model, the functional motion model simulating a dynamic musculoskeletal joint motion.

In Example 18, the subject matter of Examples 1-17 includes, generating a machine learning dynamic musculoskeletal joint model based on the first sensor data, the medical imaging data, and a plurality of musculoskeletal joint machine learning data; wherein generating the functional digital twin model includes generating a plurality of dynamic musculoskeletal joint data based on the machine learning dynamic musculoskeletal joint model.

Example 19 is one or more machine-readable medium including instructions, which when executed by a computing system, cause the computing system to perform any of the methods of Examples 1-18.

Example 20 is an apparatus comprising means for performing any of the methods of Examples 1-18.

Example 21 is a system to perform the operations of any of the methods of Examples 1-18.

Example 22 is a system for generating an arthroplasty functional digital twin, the system comprising: a first plurality of sensors attached to a patient; processing circuitry; and a memory that includes, instructions, the instructions, when executed by the processing circuitry, cause the processing circuitry to: receive first sensor data from the first plurality of sensors, the first sensor data characterizing a first musculoskeletal joint of the patient; receive medical imaging data of the first musculoskeletal joint; generate a functional digital twin model of the first musculoskeletal joint based on the first sensor data and the medical imaging data, the functional digital twin model indicating a preoperative range of motion of the first musculoskeletal joint; and output the functional digital twin model of the first musculoskeletal joint.

In Example 23, the subject matter of Example 22 includes, a second plurality of sensors, wherein the instructions further cause the processing circuitry to: receive second sensor data from the second plurality of sensors attached to the patient, the second sensor data characterizing a second musculoskeletal joint of the patient, wherein the second musculoskeletal joint is contralateral to the second musculoskeletal joint; and generate a digital mirroring model of the second musculoskeletal joint based on the second sensor data, the digital mirroring model indicating a baseline range of motion of the second musculoskeletal joint.

In Example 24, the subject matter of Example 23 includes, the instructions further cause the processing circuitry to generate a simulated digital mirroring range of motion of the first musculoskeletal joint based on the digital mirroring model.

In Example 25, the subject matter of Examples 23-24 includes, wherein the first sensor data characterizing the first musculoskeletal joint includes determining at least one of a joint position, a joint motion, a joint strain, a joint torque, or a joint torsion.

In Example 26, the subject matter of Examples 22-25 includes, the instructions further cause the processing circuitry to: receive a simulated surgical procedure selection, the simulated surgical procedure selection including at least one of a soft tissue release and a bone cut; generate a predicted range of motion of the first musculoskeletal joint based on the simulated surgical procedure selection and the functional digital twin model; and generate an indication of the predicted range of motion.

In Example 27, the subject matter of Example 26 includes, the instructions further cause the processing circuitry to: receive a confirmation of the simulated surgical procedure selection; and generate a surgical plan based on the functional digital twin model and the confirmation of the simulated surgical procedure selection.

In Example 28, the subject matter of Example 27 includes, wherein the surgical plan includes a plurality of robotic surgical steps.

In Example 29, the subject matter of Examples 27-28 includes, wherein: the surgical plan includes a plurality of telerobotic surgical steps; and the surgical plan includes a plurality of surgeon control steps, the plurality of surgeon control steps controlling a telerobotic sequence of the plurality of telerobotic surgical steps.

In Example 30, the subject matter of Examples 27-29 includes, wherein: the surgical plan includes a plurality of robotic surgical steps and a plurality of surgeon surgical steps; the plurality of surgeon surgical steps includes a plurality of soft tissue releases; and the plurality of robotic surgical steps includes a plurality of bone cut surgical steps.

In Example 31, the subject matter of Examples 22-30 includes, wherein the medical imaging data includes a plurality of images of the first musculoskeletal joint and the first plurality of sensors.

In Example 32, the subject matter of Examples 22-31 includes, wherein the first plurality of sensors is attached to a plurality of predetermined positions on the patient, the plurality of predetermined positions on the patient selected to characterize the first musculoskeletal joint.

In Example 33, the subject matter of Examples 22-32 includes, wherein: the first plurality of sensors is embedded in a flexible ring fixed around the first musculoskeletal joint; the flexible ring is formed using a radiopaque material; and the first plurality of sensors is visible in the medical imaging data.

In Example 34, the subject matter of Examples 22-33 includes, wherein: the first musculoskeletal joint includes a knee of the patient; and the first plurality of sensors is attached around the knee of the patient, around a hip of the patient, and around an ankle of the patient.

In Example 35, the subject matter of Examples 22-34 includes, wherein: the first plurality of sensors includes a plurality of piezo sensors; and the first plurality of sensors senses at least one of musculoskeletal strain, musculoskeletal torque, and musculoskeletal torsion.

In Example 36, the subject matter of Examples 22-35 includes, wherein the first sensor data includes a relative position of each of the first plurality of sensors.

In Example 37, the subject matter of Examples 22-36 includes, wherein the first sensor data includes motion data captured during a movement of the first musculoskeletal joint.

In Example 38, the subject matter of Examples 22-37 includes, wherein: the medical imaging data of the first musculoskeletal joint includes a plurality of static musculoskeletal joint positions; and generating the functional digital twin model includes generating a functional motion model, the functional motion model simulating a dynamic musculoskeletal joint motion.

In Example 39, the subject matter of Examples 22-38 includes, the instructions further cause the processing circuitry to generate a machine learning dynamic musculoskeletal joint model based on the first sensor data, the medical imaging data, and a plurality of musculoskeletal joint machine learning data; wherein generating the functional digital twin model includes generating a plurality of dynamic musculoskeletal joint data based on the machine learning dynamic musculoskeletal joint model.

Example 40 is at least one machine-readable medium including instructions that, when executed by processing circuitry, cause the processing circuitry to perform operations to implement of any of Examples 1-39.

Example 41 is an apparatus comprising means to implement of any of Examples 1-39.

Example 42 is a system to implement of any of Examples 1-39.

Example 43 is a method to implement of any of Examples 1-39.

Method examples described herein may be machine or computer-implemented at least in part. Some examples may include a computer-readable medium or machine-readable medium encoded with instructions operable to configure an electronic device to perform methods as described in the above examples. An implementation of such methods may include code, such as microcode, assembly language code, a higher-level language code, or the like. Such code may include computer readable instructions for performing various methods. The code may form portions of computer program products. Further, in an example, the code may be tangibly stored on one or more volatile, non-transitory, or non-volatile tangible computer-readable media, such as during execution or at other times. Examples of these tangible computer-readable media may include, but are not limited to, hard disks, removable magnetic disks, removable optical disks (e.g., compact disks and digital video disks), magnetic cassettes, memory cards or sticks, random access memories (RAMs), read only memories (ROMs), and the like.

## Claims

1. A method for generating an arthroplasty functional digital twin, the method comprising:
receiving first sensor data from a first plurality of sensors attached to a patient, the first sensor data characterizing a first musculoskeletal joint of the patient;
receiving medical imaging data of the first musculoskeletal joint;
generating, at a processing circuitry of a device, a functional digital twin model of the first musculoskeletal joint based on the first sensor data and the medical imaging data, the functional digital twin model indicating a preoperative range of motion of the first musculoskeletal joint; and
outputting the functional digital twin model of the first musculoskeletal joint.

2. The method of claim 1, further including:
receiving second sensor data from a second plurality of sensors attached to the patient, the second sensor data characterizing a second musculoskeletal joint of the patient, wherein the second musculoskeletal joint is contralateral to the first musculoskeletal joint; and
generating a digital mirroring model of the second musculoskeletal joint based on the second sensor data, the digital mirroring model indicating a baseline range of motion of the second musculoskeletal joint.

3. The method of claim 2, further including generating a simulated digital mirroring range of motion of the first musculoskeletal joint based on the digital mirroring model.

4. The method of claim 2, wherein the first sensor data characterizing the first musculoskeletal joint includes determining at least one of a joint position, a joint motion, a joint strain, a joint torque, or a joint torsion.

5. The method of claim 1, further including:
receiving a simulated surgical procedure selection, the simulated surgical procedure selection including at least one of a soft tissue release and a bone cut;
generating a predicted range of motion of the first musculoskeletal joint based on the simulated surgical procedure selection and the functional digital twin model; and
generating an indication of the predicted range of motion.

6. The method of claim 5, further including:
receiving a confirmation of the simulated surgical procedure selection; and
generating a surgical plan based on the functional digital twin model and the confirmation of the simulated surgical procedure selection.

7. The method of claim 6, wherein:
the surgical plan includes a plurality of telerobotic surgical steps; and
the surgical plan includes a plurality of surgeon control steps, the plurality of surgeon control steps controlling a telerobotic sequence of the plurality of telerobotic surgical steps.

8. The method of claim 6, wherein:
the surgical plan includes a plurality of robotic surgical steps and a plurality of surgeon surgical steps;
the plurality of surgeon surgical steps includes a plurality of soft tissue releases; and
the plurality of robotic surgical steps includes a plurality of bone cut surgical steps.

9. The method of claim 1, wherein the medical imaging data includes a plurality of images of the first musculoskeletal joint and the first plurality of sensors.

10. The method of claim 1, wherein:
the first plurality of sensors is embedded in a flexible ring fixed around the first musculoskeletal joint;
the flexible ring is formed using a radiopaque material; and
the first plurality of sensors is visible in the medical imaging data.

11. The method of claim 1, wherein:
the first plurality of sensors includes a plurality of piezo sensors; and
the first plurality of sensors senses at least one of musculoskeletal strain, musculoskeletal torque, and musculoskeletal torsion.

12. The method of claim 1, wherein:
the medical imaging data of the first musculoskeletal joint includes a plurality of static musculoskeletal joint positions; and
generating the functional digital twin model includes generating a functional motion model, the functional motion model simulating a dynamic musculoskeletal joint motion.

13. The method of claim 1, further including generating a machine learning dynamic musculoskeletal joint model based on the first sensor data, the medical imaging data, and a plurality of musculoskeletal joint machine learning data;
wherein generating the functional digital twin model includes generating a plurality of dynamic musculoskeletal joint data based on the machine learning dynamic musculoskeletal joint model.

14. One or more machine-readable medium including instructions, which when executed by a computing system, cause the computing system to perform any of the methods of claims 1-13.

15. An apparatus comprising means for performing any of the methods of claims 1-13.
